# EUROPÄISCHE PATENTANMELDUNG

(11) **EP 1 776 951 A1**
(43) Veröffentlichungstag der Anmeldung: **25.04.2007**
(21) Anmeldenummer: 06019316.6
(22) Anmeldetag: 15.09.2006
(51) Int. Cl.: A61K 31/045, A61P 9/00

(54) **Langkettige Alkohole zur Suppression der Konzentrationen des C-reaktiven Proteins (CRP) im menschlichen Blut**

(30) Priorität: 24.09.2005 DE 102005045829
(71) Anmelder: Clariant Produkte (Deutschland) GmbH, 65929 Frankfurt am Main (DE)
(72) Erfinder: Kammermeier, Bernhard, Dr., 80687 München (DE); Heinrichs, Franz-Leo, Dr., 86456 Gablingen (DE); Krendlinger, Ernst, Dr., 86316 Friedberg (DE)
(74) Vertreter: Paczkowski, Marcus

(57) **Zusammenfassung**

Langkettige Alkohole auf Basis von Montanwachsalkoholen, Bienenwachsalkoholen oder Guerbetalkoholen oder Mischungen daraus lassen sich vorteilhaft in einem Mittel zur effektiven Senkung erhöhter CRP-Spiegel verwenden. Dadurch wird überraschend auch eine positive Beeinflussung von pathophysiologischen Vorgängen möglich, insbesondere von KHK, von Schlaganfall oder Komplikationen des rheumatoiden Formenkreises, die mit erhöhtem CRP-Spiegel assoziiert sind.

## Beschreibung

Die Erfindung betrifft die Verwendung von langkettigen Alkoholen in einem Mittel zur effektiven Senkung pathologisch erhöhter CRP-Spiegel.

Das C-reaktive Protein (CRP) ist ein in der Leber gebildeter, kohlehydratfreier Faktor, der in Zusammenhang mit entzündlichen Prozessen, sowohl leichten, als auch schweren, lokalen, als auch generalisierten auftritt. Der Name leitet sich ab von seiner Fähigkeit, in Gegenwart von Ca-lonen mit einem Pneumokokkenpolysaccharid zu reagieren. Das CRP zählt zu den ,Akute Phase Proteinen' und kann das Komplementsystem aktivieren. In der Leber wird seine Produktion durch Interleukin 6 stimuliert (siehe "http://de.wikipedia.org/wiki/C-Reaktives-Protein").

Es ist bekannt, dass erhöhte CRP-Spiegel einen unabhängigen Risikofaktor für lebensbedrohlichen Krankheiten des kardiovaskulären Formenkreises, oder für Schlaganfall darstellen (New England Journal of Medicin, 347, 2002, 1557). Neuere Forschungen weisen CRP auch die Rolle eines Risikofaktors für Arteriosklerose zu (siehe"http://www.ilexikon.com/C-Reaktives-Protein.html").

Es ist weiter bekannt, dass Statine das CRP auch in Abwesenheit einer Hyperlipidämie senken (Journal of American Medical Association, 2002, 286: 64-70) und dass CRP möglicherweise ein stärkerer prädikativer Faktor hinsichtlich KHK-Risiko ist, als erhöhte LDL-Spiegel (siehe http://www.aerztezeitunm.de/docs/2002/11/18/208al 11.asp?cat=/medizin/cholesterin).

Zusätzlich ist bekannt, dass erhöhte CRP-Spiegel auch bei rheumatischen Krankheiten eine Rolle spielen. In der Rheumatologie ermöglicht das CRP die Unterscheidung zwischen entzündlich-rheumatischen Erkrankungen und nichtentzündlich rheumatischen Erkrankungen (siehe http//www.rheuma-online.de/a-z/c/c-reaktives-protein.html). Das CRP wird hier etwa schnell durch Cortison beeinflusst. Eine Beeinflussung der Entzündungsmarker CRP und SAA (Serum Amyloid A Protein) ist auch durch Verabfolgung von Folaten bekannt (siehe WO 03/072096).

Aufgabe der vorliegenden Erfindung war es daher, ein Mittel zu finden, mit dem sich die Senkung des CRP-Spiegels im Blut erreichen lässt und mit dem somit alle oben genannten und mit einem hohen CRP-Spiegel assoziierten pathologischen Formenkreise positiv zu beeinflussen sind, hauptsächlich auf die positive Beeinflussung von kardiovaskulären Komplikationen über eine Absenkung der Konzentration des CRP.

Gelöst wird diese Aufgabe durch die Verwendung von langkettigen Alkoholen auf Basis von Montanwachsalkoholen, Bienenwachsalkoholen und/oder Guerbetalkoholen in einem Mittel zur effektiven Senkung erhöhter CRP-Spiegel.

Für langkettige Alkohole, wie sie in den oben genannten Montanwachsalkoholen, Bienenwachsalkoholen und Guerbetalkoholen auftreten, sind Cholesterin senkende Effekte in Analogie zu Statinen bekannt (siehe E. Ernst, MMW Nr.23/2002, Seite 20, oder WO 00/78 697 A2). Tierexperimentelle Untersuchungen ergaben dabei, dass dieses alkoholische Substanzgemisch neben den genannten Effekten noch weitere antiatherogene und andere positive gesundheitliche Effekte aufweist, wobei keine adversen Effekte gefunden wurden. Vergleichsstudien zeigen eine zu herkömmlichen Statinen (verglichen wurden Simvastatin und Pravastatin) äquivalente Wirkung bei fehlendem Nebenwirkungsprofil in einem Dreijahresvergleich.

Durch die pathophysiologische Korrelation von erhöhten LDL- und erhöhten CRP-Spiegeln kann auf eine physiologische Wechselwirkung von LDL- und CRP Spiegel geschlossen werden.

Völlig überraschend wurde gefunden, dass die Verwendung von langkettigen Alkoholen auf Basis von Montanwachsalkoholen, deren Bienwachsanaloga und/oder von Guerbetalkoholen in einem Mittel zur effektiven Senkung pathologisch erhöhter CRP-Spiegel hervorragend geeignet ist und dass damit die positive Beeinflussung von mit erhöhtem CRP-Spiegel assoziierten pathophysiologischen Vorgängen möglich wird.

Von den vorgenannten langkettigen Alkoholen werden keine speziellen Alkoholfraktionen abgetrennt, sondern es werden die ggf. vorgereinigten jeweiligen Gesamtalkoholanteile verwendet.

Vorteilhafter Weise zeigen die aus den o.g. Wachsalkoholen abzuleitenden langkettigen unverzweigten Alkohole sowie die Guerbetalkohole allein oder im Gemisch miteinander eine weit einfachere, kostengünstigere bis teilweise auch stärkere Wirkung im Hinblick auf die Suppression des CRP-Spiegels, als die bekannten Statine.

Die genannten Wachsalkohole erweisen sich als langkettige, unverzweigte bzw. verzweigte Alkohole in gewerbetoxikologischen Studien zusätzlich als untoxisch (siehe: Artikel "Montan Wax". In: Ullmann's Encyclopedia of Industrial Chemistry, Vol A 28, 5. Auflage, Weinheim, Verlag Chemie, S.122-126). Der große Vorteil der genannten Alkohole liegt in Ihrer einfachen großtechnischen Synthese und in ihrer qualitativen Reproduzierbarkeit und Verfügbarkeit.

Die Alkohole können durch Verseifungsreaktionen vom Wachs gespalten und anschließend reinigend extrahiert werden. Das Montanwachs kann auch zunächst oxidativ, z.B. mit Chromschwefelsäure (sog. Gersthofen Prozess), gespalten werden und die resultierenden Montanwachssäuren können danach reduktiv in Alkohole überführt werden. Guerbetalkohole sind gleichfalls aus gut zugänglichen Rohstoffen durch eine sehr einfache chemische Umsetzung zugänglich.

Die langkettigen Alkohole werden erfindungsgemäß in Mengen im Bereich von 0,01 bis 3 Gew.-% eingesetzt, bezogen auf das Körpergewicht des Patienten, vorzugsweise von 0,1 bis 2 Gew.-%. Die Applikation erfolgt dabei vorzugsweise oral, sie kann aber auch bei entsprechender Galenik rektal, subkutan oder intravenös erfolgen.

## Patentansprüche

1. Verwendung von langkettigen Alkoholen auf Basis von Montanwachsalkoholen, von Bienenwachsalkoholen und/oder von Guerbetalkoholen in einem Mittel zur effektiven Senkung erhöhter CRP-Spiegel.

2. Verwendung nach Anspruch 1, **dadurch gekennzeichnet, dass** dadurch eine positive Beeinflussung von pathophysiologischen Vorgängen, insbesondere von koronarer Herzkrankheit, von Schlaganfall oder Komplikationen des rheumatoiden Formenkreises bewirkt wird, die mit erhöhtem CRP-Spiegel assoziiert sind.

3. Verwendung nach Anspruch 1 oder 2, **dadurch gekennzeichnet, dass** die langkettigen Alkohole als gewerbetoxikologisch unbedenkliche Strukturen zur Vermeidung von Rhabdomyelose Komplikationen, wie sie von Statinen bekannt sind, verwendet werden.

4. Verwendung nach einem oder nach mehreren der Ansprüche 1 bis 3, **dadurch gekennzeichnet, dass** zusätzlich eine einfache und gut etablierte Analytik zur Bestimmung des Produktgehaltes an Montanwachsalkoholen bzw. Guerbetalkoholen eingesetzt wird.

5. Verwendung nach einem oder nach mehreren der Ansprüche 1 bis 4, **dadurch gekennzeichnet, dass** durch die Verwendung der langkettigen Alkohole keine kalorische Zusatzbelastung auftritt.

6. Verwendung nach einem oder nach mehreren der Ansprüche 1 bis 5, **dadurch gekennzeichnet, dass** die langkettigen Alkohole in Mengen im Bereich von 0,01 bis 3 Gew.-%, bezogen auf das Gewicht des Patienten eingesetzt werden.

7. Verwendung nach einem oder nach mehreren der Ansprüche 1 bis 6, **dadurch gekennzeichnet, dass** die langkettigen Alkohole in einem Mittel zur oralen Applikation verwendet werden.
